# EUROPEAN PATENT SPECIFICATION

(11) **EP 4 692 694 B1**
(45) Date of publication and mention of the grant of the patent: **21.10.2026**
(21) Application number: 24838586.6
(22) Date of filing: 25.06.2024
(51) Int. Cl.: A01N 1/145, C12N 5/00, F25D 3/10, F25D 17/06, F25D 23/00, F25D 23/02, F25D 23/12, F25D 25/04, F25D 29/00

(54) **BIOLOGICAL SAMPLE COOLING DEVICE AND MULTIFUNCTIONAL COOLING SYSTEM**
VORRICHTUNG ZUR KÜHLUNG BIOLOGISCHER PROBEN UND MULTIFUNKTIONELLES KÜHLSYSTEM
DISPOSITIF DE REFROIDISSEMENT D'ÉCHANTILLON BIOLOGIQUE ET SYSTÈME DE REFROIDISSEMENT MULTIFONCTIONNEL

(30) Priority: 10.07.2023 CN 202310835987
(43) Date of publication of application: 11.02.2026
(73) Proprietor: SHANGHAI ORIGINCELL BIOLOGICAL CRYO EQUIPMENT CO., LTD., Shanghai 201203 (CN)
(72) Inventor: QU, Jianguo, Shanghai 201203 (CN); LIU, Zhikang, Shanghai 201203 (CN)
(74) Representative: Bayramoglu et al.
(86) International application number: PCT/CN2024/101305
(87) International publication number: WO 2025/011330

(56) References cited:
- CN-A- 111 536 732
- CN-A- 116 951 858
- CN-U- 214 853 921
- CN-U- 214 853 921
- CN-U- 215 571 512
- CN-U- 217 509 762
- CN-U- 217 509 762
- US-A1- 2006 204 950
- US-A1- 20240 196 884
- ANONYMOUS: "The next generation biobank system", 1 October 2020 (2020-10-01), Internet, XP093194949, Retrieved from the Internet <URL:https://labplan.ie/content/uploads/2019/10/Askion-2020-Catalogue.pdf> [retrieved on 20260528]

## Description

### Technical field

The present invention relates to the technical field of biological sample storage, in particular to a biological sample cooling device and a multi-functional cooling system.

### Background

At present, the automatic cooling function of cryopreservation tubes and cryopreservation boxes for biological samples cannot be achieved. The biological samples typically need to be stored at ultra-low temperature environments, such as an storage temperature generally including -80°C, -140°C, -196°C, etc., while the cryopreservation speed of the biological sample plays an important role in the activity of the biological sample. The conventional storage method is that the cryopreservation box in a transfer case or a transfer tank is directly put in the tank body, but a certain slow gradient cooling action is not achieved, which easily causes a risk that the biological sample is damaged due to too quick cooling.

Therefore, the inventor invents a device for slowly cooling the biological sample, which can heat a gas in a cabin body when the temperature of liquid nitrogen is too low so that the cooled environment is warmed up, and performs cooling storage on multiple biological sample grillages. CN214853921U discloses a sample cooling storage mechanism. The sample cooling storage mechanism comprises refrigeration equipment and gradient cooling equipment. The refrigeration device can control the temperature of the cryopreservation tubes for refrigeration; the gradient cooling device can perform gradient programmed cooling on the cryopreservation tubes; the refrigeration device includes at least one refrigeration zone; the refrigeration zone is equipped with a nitrogen spraying component, a heating component, and a cryopreservation tube rack. The nitrogen spraying component can spray nitrogen in the refrigeration zone, and the heating component can heat the temperature inside the refrigeration zone. CN217509762U discloses a sample program cooling equipment, including low temperature storage box, extraction system and transfer system, the extraction system is provided at the upper end of low temperature storage box and can carry out extraction operation in low temperature storage box, the transfer system is provided at the position corresponding to the extraction system, and the transfer system is provided with the extraction system. The extraction system is provided with a sealed receiving opening; the conveying system comprises a conveying opening, a conveying rail, a jacking component and a conveying plate trailer. The jacking component is arranged at the position corresponding to the sealed receiving opening. The conveying track is arranged between the conveying opening and the jacking component; a jacking receiving plate is arranged on the upper end face of the jacking component, and the conveying plate trailer is arranged on the conveying rail in a sliding mode and can slide to the jacking receiving plate from the conveying rail.

### Summary

The objective of this section is to summarize some aspects of the embodiments of the present invention and briefly introduce some preferred embodiments. In this section and the Abstract and invention name of the present application, some simplifications or omissions may be made to avoid the objectives of this section, the Abstract and invention name to become fuzzy, and such the simplifications or omissions cannot be used for limiting the scope of the present invention.

In view of the problems as described above or existing in the prior art, the present invention is proposed.

Therefore, the objective of the present invention is to provide a biological sample cooling device. The biological sample cooling device can cool biological samples in a biological grillage, i.e., slow gradient cooling can be performed on the biological samples through a cooling adjusting assembly; and when the too low temperature of liquid nitrogen is detected through a circulation cooling assembly, a gas is sucked, heated and then fed into a circulation box to be mixed with liquid nitrogen so as to increase the temperature of liquid nitrogen, so that the biological sample is slowly cooled, and multiple groups of biological samples can be stored in the circulation box.

In order to solve the above technical problems, the present invention provides the following technical solution: a biological sample cooling device, comprising a cavity assembly, a cooling adjusting assembly, a circulation cooling assembly and biological grillages, wherein
the cavity assembly is provided with the cooling adjusting assembly and the circulation cooling assembly, the circulation cooling assembly is arranged at the side of the cooling adjusting assembly, and the cooling adjusting assembly is configured to individually perform gradient cooling on the biological grillages; multiple groups of the biological grillages are accommodated inside the circulation cooling assembly, and the circulation cooling assembly is configured to perform adjustable temperature control on the multiple groups of the biological grillages inside the circulation cooling assembly; the circulation cooling assembly comprises a circulation barrel member, an air suction member and a heating member, wherein the circulation barrel member is connected and fixed with the cavity assembly, the lower end of the circulation barrel member is configured to communicate with the heating member through a pipeline, the heating member is configured to communicate with the air suction member, and the air suction member is connected with the cavity assembly, such that a gas inside the cavity assembly is sucked by the air suction member and heated by the heating member, then the heated gas and liquid nitrogen are mixed in the circulation barrel member so that the temperature of liquid nitrogen increases, thereby avoiding quick cooling.

As a preferred embodiment of the biological sample cooling device of the present invention, the cooling adjusting assembly comprises a cooling barrel member and a lifting and accessing member, wherein
the upper part of the cooling barrel member is provided with the lifting and accessing member, the lifting and accessing member is configured to drive the biological grillages to enter the cooling barrel member, the cooling barrel member is internally provided with liquid nitrogen, the cooling barrel member is configured to control the temperature of the liquid nitrogen, and the lifting and accessing member is configured to detect the temperatures of the biological grillages and the environment temperature of the biological grillages.

As a preferred embodiment of the biological sample cooling device of the present invention, the cooling barrel member comprises a cooling vacuum barrel, a liquid nitrogen valve and a liquid adding pipeline, wherein
the cooling vacuum barrel is connected and fixed with the cavity assembly, and the liquid nitrogen valve is configured to control the liquid adding pipeline to place the liquid nitrogen into the cooling vacuum barrel.

As a preferred embodiment of the biological sample cooling device of the present invention, the lifting and accessing member comprises a support plate, a support sliding plate, a guide rail support plate, a lead screw, a landing slider, a fixed block, a buffer spring, a temperature sensor, a sample temperature detecting tube, a grillage storage platform, a first motor and a slider guide rail, wherein
the side surface of the support plate is provided with the support sliding plate, the side surface of the support sliding plate is connected with the guide rail support plate, the side surface of the upper end of the guide rail support plate is connected with a first motor support platform, the first motor support platform is equipped with the first motor, the first motor is vertically connected with the lead screw through a transmission shaft, the lead screw is connected with the fixed block and the landing slider, the side surface of the guide rail support plate is fixedly provided with the slider guide rail, the landing slider and the fixed block are in sliding connection with the slider guide rail, the lower end of the fixed block is connected with the buffer spring, the lower end of the buffer spring is connected with the temperature sensor, the lower end of the temperature sensor is provided with the sample temperature detecting tube, the lower end of the guide rail support plate is connected with the grillage storage platform, and the grillage storage platform is configured to be used for placing the biological grillages; and the side surface of the support sliding plate is connected with chains, and the support sliding plate is in up and down sliding connection with the support plate.

As a preferred embodiment of the biological sample cooling device of the present invention, the air suction member comprises an air blower, a mesh plate and an air suction tube, wherein
the upper part of the air blower is connected with the mesh plate, and the air blower is configured to communicate with the air suction tube.

As a preferred embodiment of the biological sample cooling device of the present invention, the heating member comprises a heating module and an air inlet tube, wherein
the heating module is configured to communicate with the air suction tube, and the heating module is configured to communicate with the air inlet tube.

As a preferred embodiment of the biological sample cooling device of the present invention, the circulation barrel member comprises a docking cavity, nitrogen injection tubes, gas ejector tubes, a guided cooling dish and a circulation barrel, wherein
the air inlet tube is configured to communicate with the docking cavity, the docking cavity is arranged inside the circulation barrel, the nitrogen injection tubes are arranged around the docking cavity and configured to communicate with the docking cavity, the gas ejector tubes are arranged on the upper parts of the nitrogen injection tubes, the gas ejector tubes are configured to communicate with the docking cavity through pipelines, multiple groups of the gas ejector tubes and the nitrogen injection tubes are circumferentially arranged, the gas ejector tubes are upwardly provided with round holes, the upper parts of the gas ejector tubes are provided with the guided cooling dish, the guided cooling dish is provided with meshed-shaped holes, the upper part of the guided cooling dish is provided with grillage columns, and the biological grillages are placed inside the circulation barrel through the grillage columns; multiple groups of the biological grillages are arranged inside the circulation barrel.

As a preferred embodiment of the biological sample cooling device of the present invention, the nitrogen injection tube is configured to communicate with the liquid adding pipeline.

The biological sample cooling device of the present invention has the following beneficial effects: slow gradient cooling can be performed on a whole-plate biological sample through the cooling adjusting assembly; the temperature of liquid nitrogen is detected by the circulation cooling assembly; when the temperature is too low, a gas in the cavity assembly is sucked into the heating module to be heated, so that the liquid nitrogen is warmed up, thereby achieving intelligent temperature adjustment; furthermore, multiple groups of biological samples are stored in the circulation cooling assembly for common temperature adjustment.

In view of a fact that it is often needed for placing the biological grillages in the circulation barrel during the use, the grillage grabbing assembly is arranged; and since the biological grillages outside are inconveniently placed in the operating cavity, the transfer tank is arranged to be docked with the cavity assembly. Based on this, the inventor invents a multi-functional cooling system.

In order to solve the above technical problem, the present invention also provides the following technical solution: a multi-functional cooling system, comprising a biological sample cooling device, and also comprising a transport assembly, a lifting assembly, a support rack assembly, a cover opening assembly, a rotatory liquid adding assembly, a grillage grabbing assembly, a pipette assembly, a sample temporary storage box, a barcode scanner and a transfer tank, wherein
the support rack assembly is configured to support the transport assembly, the lifting assembly and the cavity assembly, the transport assembly is configured to transport the transfer tank, the lifting assembly is configured to lift up the transfer tank and to be docked with the cavity assembly, the cover opening assembly is configured to open an upper cover of the transfer tank, the rotatory liquid adding assembly is configured to add the liquid into the transfer tank, the pipette assembly is configured to suck redundant nitrogen, the grillage grabbing assembly is configured to be used for grabbing the biological grillages, the sample temporary storage box is configured to be used for placing the biological grillages, and the barcode scanner is configured to scan the labels of the biological grillages.

As a preferred embodiment of the multi-functional cooling system of the present invention, the cavity assembly comprises an operating support plate and an operating cavity housing, wherein the upper part of the operating support plate is connected with the operating cavity housing, the operating support plate and the operating cavity housing form an operating cavity, and the operating cavity is internally provided with the cover opening assembly, the rotatory liquid adding assembly, the grillage grabbing assembly, the pipette assembly, the sample temporary storage box and the barcode scanner.

As a preferred embodiment of the multi-functional cooling system of the of the present invention, the support rack assembly comprises a support rack and a support platform, wherein the upper end of the support rack is connected with the operating support plate, and the side end of the support rack is provided with the support platform.

As a preferred embodiment of the multi-functional cooling system of the present invention, the transport assembly comprises a transport vehicle and a transport guide rail, wherein
the transport guide rail is arranged on the upper part of the support platform, and the transport vehicle is in sliding connection with the transport guide rail.

As a preferred embodiment of the multi-functional cooling system of the present invention, the lifting assembly comprises a lifting guide rail, a lifting motor, a lifting lead screw, a lifting slider, a lifting U-shaped plate and a lifting plate, wherein
the lifting guide rail is vertically and fixedly connected with the support rack, the lifting slider is in cooperative connection with the lifting lead screw, the upper end and lower end of the lifting guide rail have bumps for supporting the lifting lead screw, the lifting motor is configured to drive the lifting lead screw to rotate through a belt wheel, the side surface of the lifting slider is fixedly connected with the lifting U-shaped plate, the lifting plate is arranged at the upper side of the middle of the transport vehicle, the transfer tank is arranged on the upper end surface of the lifting plate, and the lifting plate is configured to rise and fall through the lifting U-shaped plate.

As a preferred embodiment of the multi-functional cooling system of the present invention, the cover opening assembly comprises a door cover plate, a door cover motor and a door cover spindle, wherein
the door cover motor is connected with the door cover spindle, the door cover plate is connected with the door cover spindle, the door cover spindle is configured to perform supporting through a door cover support base, and the door cover support base is connected with the operating support plate.

As a preferred embodiment of the multi-functional cooling system of the present invention, the grillage grabbing assembly comprises X-axis sliding rails, Y-axis sliding rails, a gripper motor, a gripper spindle and grillage grippers, wherein
the Y-axis sliding rails are connected with the inner side of the operating cavity housing, the Y-axis sliding rails are symmetrically arranged, two groups of symmetrical Y-axis sliding rails are connected through the X-axis sliding rails, the X-axis sliding rails are in sliding connection with the Y-axis rails, one sides of the X-axis sliding rails are provided with the gripper motor, the gripper motor is connected with the gripper spindle through the belt wheel, and the lower part of the gripper spindle is connected with the grillage grippers.

As a preferred embodiment of the multi-functional cooling system of the present invention, the pipette assembly comprises an electric cylinder, a pipette motor, a liquid nitrogen cup, a pipette lead screw, a pipette and a pipette tip, wherein
the electric cylinder is arranged at one sides of the X-axis sliding rails far away from the gripper motor, the side surface of the electric cylinder is provided with a motor guide rail, the pipette motor in sliding connection with the motor guide rail and the pipette lead screw connected with the pipette motor, the lower part of the pipette motor is connected with the pipette, the outer side of the pipette is provided with the liquid nitrogen cup, and the lower end of the pipette is connected with the pipette tip.

As a preferred embodiment of the multi-functional cooling system of the present invention, the sample temporary storage box is arranged on the operating support plate, the biological grillages are stored in the sample temporary storage box, the upper part of the sample temporary storage box is provided with a second door cover plate, and a second door cover plate motor is configured to drive the second door cover plate to open or close the sample temporary storage box.

As a preferred embodiment of the multi-functional cooling system of the present invention, the rotatory liquid adding assembly comprises a rotatory liquid adding motor, a liquid adding barrel and a liquid supplementing tube, wherein
the rotatory liquid adding motor is connected with the liquid adding barrel through the belt wheel, the liquid supplementing tube is connected with the liquid adding barrel, the liquid adding barrel is configured to perform supporting through the support shaft, and the support shaft is connected with the inner side surface of the operating cavity housing.

The multi-functional cooling system of the present invention has the following beneficial effects: the transfer tank can move above the lifting assembly through the transport assembly, the lifting assembly drives the transfer tank to rise to be docked with the cavity assembly, after being docked, the cover opening assembly can open the upper cover of the transfer tank to grab the biological grillage or biological single-tube sample by utilizing the grillage grabbing assembly and the pipette assembly, thereby putting the biological grillage or biological single-tube sample into the cooling adjusting assembly or the circulation cooling assembly, so as to achieve the cooling of the biological sample.

### Brief Description of the Drawings

To more clearly explain the technical solutions of the embodiments of the present invention, drawings required to be used in the descriptions of the embodiments will be simply introduced, obviously, the drawings in the following description are merely some embodiments of the present invention, persons of ordinary skill in the art can also obtain other drawings according to these drawings without creative efforts.
FIG. 1 is an overall diagram of a biological sample cooling device.
FIG. 2 is a diagram of a hidden operating cavity housing of a biological sample cooling device.
FIG. 3 is a stereoscopic diagram of a cooling adjusting assembly and a circulation cooling assembly of a biological sample cooling device.
FIG. 4 is an enlarged view of a biological sample cooling device at F1.
FIG. 5 is a disassembled view of a circulation cooling assembly of a biological sample cooling device.
FIG. 6 is an enlarged view of a biological sample cooling device at F2.
FIG. 7 is a stereoscopic diagram of a multi-functional cooling system.
FIG. 8 is a diagram of a multi-functional cooling system in another perspective.
FIG. 9 is a diagram of a rotatory liquid adding assembly of a multi-functional cooling system.
FIG. 10 is a diagram of a pipette assembly of a multi-functional cooling system.
FIG. 11 is a diagram of a grillage grabbing assembly of a multi-functional cooling system.
FIG. 12 is a diagram of a lifting assembly of a multi-functional cooling system.

Reference numbers: cavity assembly, 1; cooling adjusting assembly, 2; circulation cooling assembly, 3; biological grillage, 15; cooling barrel member, 21; lifting and accessing member, 22; cooling vacuum barrel, 211; liquid nitrogen valve, 212; liquid adding pipeline, 213; support plate, 221; support sliding plate, 222; guide rail support plate, 223; lead screw, 224; landing slider, 225; fixed block, 226; buffer spring, 227; temperature sensor, 228; sample temperature detecting tube, 229; grillage storage platform, 230; first motor, 231; slider guide rail, 232; circulation barrel member, 31; air suction member, 33; heating member, 35; air blower, 331; mesh plate, 332; air suction tube, 333; heating module, 351; air inlet tube, 352; docking cavity, 311; nitrogen injection tube, 312; gas ejector tube, 313; guided cooling dish, 314; circulation barrel, 315; transport assembly, 4; lifting assembly, 5; support rack assembly, 6; cover opening assembly, 7; rotatory liquid adding assembly, 8; grillage grabbing assembly, 9; pipette assembly, 10; sample temporary storage box, 11; barcode scanner, 12; transfer tank, 13; operating support plate, 111; operating cavity housing, 112; support rack, 61; support platform, 62; transport vehicle, 41; transport guide rail, 42; lifting guide rail, 51; lifting motor, 52; lifting lead screw, 53; lifting slider, 54; lifting U-shaped plate, 55; lifting plate, 56; door cover plate, 71; door cover motor, 72; door cover spindle, 73; X-axis sliding rail, 911; Y-axis sliding rail, 912; gripper motor, 913; gripper spindle, 914; grillage gripper, 915; pipette motor, 102; liquid nitrogen cup, 104; pipette lead screw, 105; pipette, 106; pipette tip, 107; rotatory liquid adding motor, 81; liquid adding barrel, 82; and liquid supplementing tube, 83.

### Detailed description of the embodiments

To make the above objective, features and advantages of the present invention more obvious and understandable, the specific embodiments of the present invention will be described in detail with reference to the drawings.

Many specific details will be set forth in the following description so as to sufficiently understand the present invention, however, the present invention can also be implemented by using other methods different from those described herein, those skilled in the art can make similar promotion without prejudice to the content of the present invention, and therefore the present invention is not limited by specific embodiments disclosed below.

Next, "one embodiment" or "embodiment" herein refers to specific features, structures or characteristics contained in at least one implementation mode of the present invention. "In one embodiment" occurring in different places of this specification does not refer to the same embodiment, nor are separate or selective embodiments mutually exclusive of other embodiments.

### Example 1

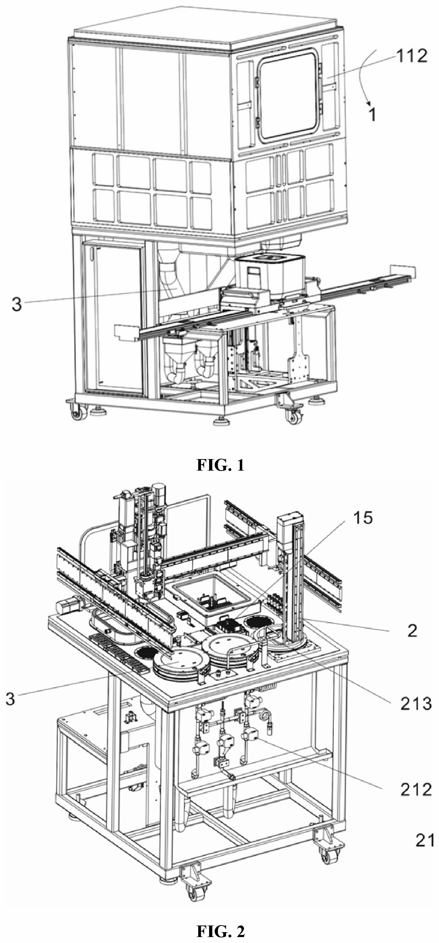

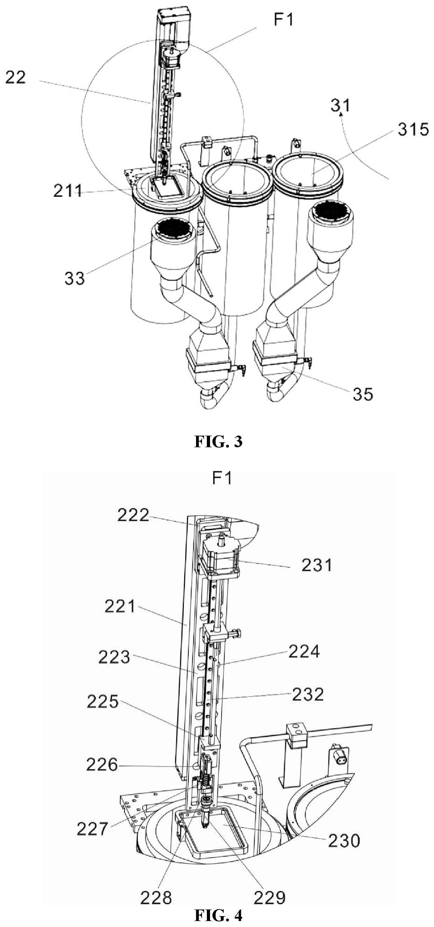

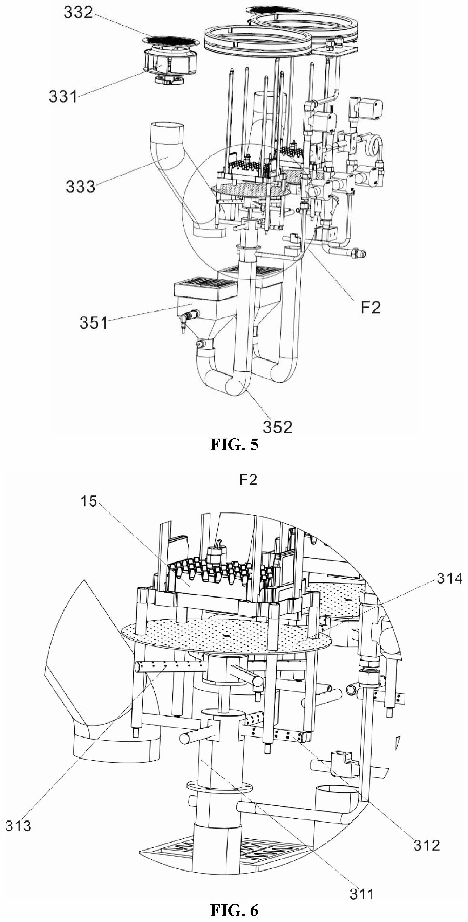

Referring to FIG. 1-FIG. 6, showing the first example of the present invention. This example provides a biological sample cooling device, comprising a cavity assembly 1, a cooling adjusting assembly 2, a circulation cooling assembly 3 and biological grillages 15, wherein a closed operating cavity is formed inside the cavity assembly 1 by arranging the cavity assembly 1, gradient cooling is performed on a single biological grillage 15 through the circulation cooling assembly 3, multiple single-tube biological samples are placed on the biological grillage 15, the temperature of liquid nitrogen can be detected by the circulation cooling assembly 3, if the temperature is too low, a gas in the cavity assembly 1 is sucked into the heating module to be heated and then mixed with liquid nitrogen, so as to increase the temperature of liquid nitrogen to slowly cool the biological sample, thereby achieving intelligent temperature adjustment; and multiple groups of biological grillages 15 can be stored in the circulation cooling assembly 3, thereby greatly improving the working efficiency.

Specifically, the biological sample cooling device comprises the cavity assembly 1, the cooling adjusting assembly 2, the circulation cooling assemblies 3 and the biological grillages 15, wherein
the cavity assembly 1 is provided with the cooling adjusting assembly 2 and the circulation cooling assembly 3, the circulation cooling assembly 3 is arranged at the side of the cooling adjusting assembly 2, and the cooling adjusting assembly 2 individually performs gradient cooling on the biological grillages 15; and multiple groups of biological grillages 15 can be accommodated inside the circulation cooling assembly 3, and the circulation cooling assembly 3 performs adjustable temperature control on the multiple groups of biological grillages 15 inside the circulation cooling assembly 3; and at least one group of cooling adjusting assembly 2 and/or circulation cooling assembly 3 is arranged.

Preferably, multiple groups of circulation cooling assemblies 3 can be arranged, in such the way, more biological samples can be stored.

Further, the cooling adjusting assembly 2 comprises a cooling barrel member 21 and a lifting and accessing member 22, wherein
the upper part of the cooling barrel member 21 is provided with the lifting and accessing member 22, the lifting and accessing member 22 drives the biological grillages 15 to enter the cooling barrel member 21, the cooling barrel member 21 is internally provided with liquid nitrogen, the cooling barrel member 21 controls the temperature of the liquid nitrogen, and the lifting and accessing member 22 can detect the temperatures of the biological grillages 15 and the environment temperature of the biological grillages 15.

Preferably, an operating support plate 111 is provided with a notch, the cooling barrel member 21 is arranged in the notch, and the lifting and accessing member 22 can drive the biological grillages 15 to rise and fall.

Further, the cooling barrel member 21 comprises a cooling vacuum barrel 211, a liquid nitrogen valve 212 and a liquid adding pipeline 213, wherein
the cooling vacuum barrel 211 is connected and fixed with the cavity assembly 1, and the liquid nitrogen valve 212 can control the liquid adding pipeline 213 to place liquid nitrogen into the cooling vacuum barrel 211.

Preferably, the cooling vacuum barrel 211 is fixed with the operating support plate 111, and liquid nitrogen flows into the cooling vacuum barrel 211 through the liquid adding pipeline 213.

Further, the lifting and accessing member 22 comprises a support plate 221, a support sliding plate 222, a guide rail support plate 223, a lead screw 224, a landing slider 225, a fixed block 226, a buffer spring 227, a temperature sensor 228, a sample temperature detecting tube 229, a grillage storage platform 230, a first motor 231 and a slider guide rail 232, wherein
the side surface of the support plate 221 is provided with the support sliding plate 222, the side surface of the support sliding plate 222 is connected with the guide rail support plate 223, the side surface of the upper end of the guide rail support plate 223 is fixedly connected with a first motor support platform, the first motor support platform is equipped with the first motor 231, the first motor 231 is vertically connected with the lead screw 224 through a transmission shaft, the lead screw 224 is in cooperative connection with the landing slider 225, the fixed block 226 is fixedly connected with the landing slider 225, the side surface of the guide rail support plate 223 is fixedly provided with the slider guide rail 232, the landing slider 225 and the fixed block 226 are in sliding connection with the slider guide rail 232, the lower end of the fixed block 226 is connected with the buffer spring 227, the lower end of the buffer spring 227 is connected with the temperature sensor 228, the lower end of the temperature sensor 228 is provided with the sample temperature detecting tube 229, the lower end of the guide rail support plate 223 is connected with the grillage storage platform 230, and the grillage storage platform 230 is used for placing the biological grillages 15; and the side surface of the support sliding plate 222 is connected with chains, the chains can drive the support sliding plate 222 to up and down move, and the support sliding plate 222 is in up and down sliding connection with the support plate 221.

Preferably, the support plate 221 is fixedly connected with the inner side of the operating cavity housing 112, and the support sliding plate 222 slides up and down sliding along a rail located on the side surface of the support plate 221.

The movement process of the lifting and accessing member 22 is as follows: the motor drives the chains to drive the support sliding plate 222 to up and down move along the rail located on the side surface of the support plate 221 so that the support sliding plate 222 is driven to move; and the support sliding plate 222 can drive the first motor 231 to move, and the grillage storage platform 230 fixed with the support sliding plate 222 can also move at the same time; and the biological grillages 15 on the grillage storage platform 230 can also move, during the downward movement, the biological grillages 15 are driven to enter the inside of the cooling vacuum barrel 211, the first motor 231 can drive the lead screw 224 to rotate, the lead screw 224 drives the fixed block 226 to move up and down, the fixed block 226 drives the temperature sensor 228 and the sample temperature detecting tube 229 to move, the cooling vacuum barrel can control the reduced temperature of liquid nitrogen and meanwhile the sample temperature detecting tube 229 can detect the temperatures of biological tubes in the biological grillages 15, and the temperature sensor 228 can detect the temperature of liquid nitrogen.

It should be noted that when the biological samples in the biological grillages 15 are put in the cooling vacuum barrel 211, the cooling vacuum barrel 211 reduces the temperatures of the biological samples by adjusting the environment temperature of the liquid nitrogen, specifically, the temperature of the biological sample is reduced from room temperature 20°C to 4°C; then, the temperature of the sample is reduced to -4°C at the rate of 1°C/min, subsequently the environment temperature is reduced to -40°C at the rate of 20°C/min, then the environment temperature is reduced to -20°C at the rate of 15°C/min, then the environment temperature is reduced to -40°C at the rate of 1°C/min and finally the environment temperature reduced to -90°C at the rate of 15°C/min, at this moment, the cooling is ended, and the activity of the biological sample is ensured through step-by-step gradient cooling.

The circulation cooling assembly 3 comprises a circulation barrel member 31, an air suction member 33 and a heating member 35, wherein
the circulation barrel member 31 is connected and fixed with the cavity assembly 1, the lower end of the circulation barrel member 31 communicates with the heating member 35 through a pipeline, the heating member 35 communicates with the air suction member 33, and the air suction member 33 is connected with the cavity assembly 1.

A gas inside the cavity assembly 1 is sucked by the air suction member 33 and heated by the heating member 35, then the heated gas and liquid nitrogen are mixed in the circulation barrel member 31 so that the temperature of liquid nitrogen increases, thereby avoiding quick cooling.

Further, the air suction member 33 comprises an air blower 331, a mesh plate 332 and an air suction tube 333, wherein
the upper part of the air blower 331 is connected with the mesh plate 332, and the air blower 331 communicates with the air suction tube 333.

Preferably, the operating support plate 111 is provided with a hole accommodating the mesh plate 332 which can filter out impurities with large sizes.

It should be noted that multiple groups of circulation cooling assemblies 3 can be arranged, in such the way, more biological samples can be stored, and the cooling of the biological sample is slowly and intelligently controlled.

Further, the heating member 35 comprises a heating module 351 and an air inlet tube 352, wherein
the heating module 351 communicates with the air suction tube 333, and the heating module 351 communicates with the air inlet tube 352.

Preferably, the gas sucked by the air suction tube 333 is heated by the heating module 351 and then fed into the air inlet tube 352.

Further, the circulation barrel member 31 comprises a docking cavity 311, nitrogen injection tubes 312, gas ejector tubes 313, a guided cooling dish 314 and a circulation barrel 315, wherein

the air inlet tube 352 communicates with the docking cavity 311, the docking cavity 311 is arranged inside the circulation barrel 315, the nitrogen injection tubes 312 are arranged around the docking cavity 311 and communicate with the docking cavity 311, the gas ejector tubes 313 are arranged on the upper parts of the nitrogen injection tubes 312, the gas ejector tubes 313 communicate with the docking cavity 311 through pipelines, multiple groups of gas ejector tubes 313 and nitrogen injection tubes 312 are circumferentially arranged, the gas ejector tubes 313 are upwardly provided with round holes, the upper parts of the gas ej ector tubes 313 are provided with the guided cooling dish 314, the guided cooling dish 314 is provided with meshed-shaped holes, the upper part of the guided cooling dish 314 is provided with grillage columns, and the biological grillages 15 are placed inside the circulation barrel 315 through the grillage columns; and multiple groups of biological grillages 15 are arranged inside the circulation barrel 315.

Preferably, multiple groups of nitrogen injection tubes 312 and gas ejector tubes 313 can be arranged, the docking cavity 311 can be fixedly connected with the air inlet tube 352, or the docking cavity 311 rotates around the air inlet tube 352, in such the way, the nitrogen injection tubes 312 can rotate and the gas ejector tubes 313 can also rotate, the nitrogen injection tubes 312 and the gas ejector tubes 313 are provided with round holes so as to facilitate the upward and more even spraying of nitrogen and the heated gas.

Further, the nitrogen injection tubes 312 communicate with the liquid adding pipeline 213.

When in use, the cooling process of the cooling adjusting assembly 2 is as follows: the motor drives the chains to drive the support sliding plate 222 to up and down move along the rail located on the side surface of the support plate 221 so as to drive the movement of the support sliding plate 222, the support sliding plate 222 can drive the first motor 231 to move, and the grillage storage platform 230 fixed with the support sliding plate 222 can also move at the same time, the biological grillages 15 stored on the grillage storage platform 230 can also move, during the downward movement, the biological grillages 15 are driven to enter the cooling vacuum barrel 211, the first motor 231 can drive the lead screw 224 to rotate, the lead screw 224 drives the fixed block 226 to up and down move, the fixed block 226 drives the temperature sensor 228 and the sample temperature detecting tube 229 to move, the cooling vacuum barrel can control the reduced temperature of liquid nitrogen and meanwhile the sample temperature detecting tube 229 can detect the temperatures of the biological tubes in the biological grillages 15, and the temperature sensor 228 can detect the temperature of liquid nitrogen.

The cooling movement of the circulation cooling assembly 3 is as follows: when a too low temperature of liquid nitrogen is detected, the gas inside the cavity assembly 1 is sucked by the air blower 331 to enter the heating module 351 via the air suction tube 333, the heated gas continues to pass through the air inlet tube 352 and the docking cavity 311 to enter the circulation barrel 315, the heated gas is ejected out through the gas ejector tube 313 and mixed with liquid nitrogen ejected from the nitrogen injection tube 312, the gas ejector tube 313 can also heat the guided cooling dish 314 located above, multiple groups of biological grillages 15 are stored on the upper part of the guided cooling dish 314 so as to perform heating treatment on the liquid nitrogen environment, thereby intelligently adjusting the cooling and ensuring the slow cooling of the biological sample.

In summary, through the cooling adjusting assembly 2, slow gradient cooling can be performed on the whole-plate biological sample, and the temperature of liquid nitrogen and the temperature of the biological sample can also be detected, thereby preventing liquid nitrogen from too quickly cooling the biological sample to inactivate the sample; and through the circulation cooling assembly 3, the temperature of liquid nitrogen can be detected, when the temperature is too low, the gas in the cavity assembly 1 is sucked into the heating module to be heated so that the liquid nitrogen is warmed up, thereby achieving intelligent temperature adjustment, and furthermore multiple groups of biological samples can be stored in the circulation cooling assembly 3 for common temperature adjustment.

### Example 2

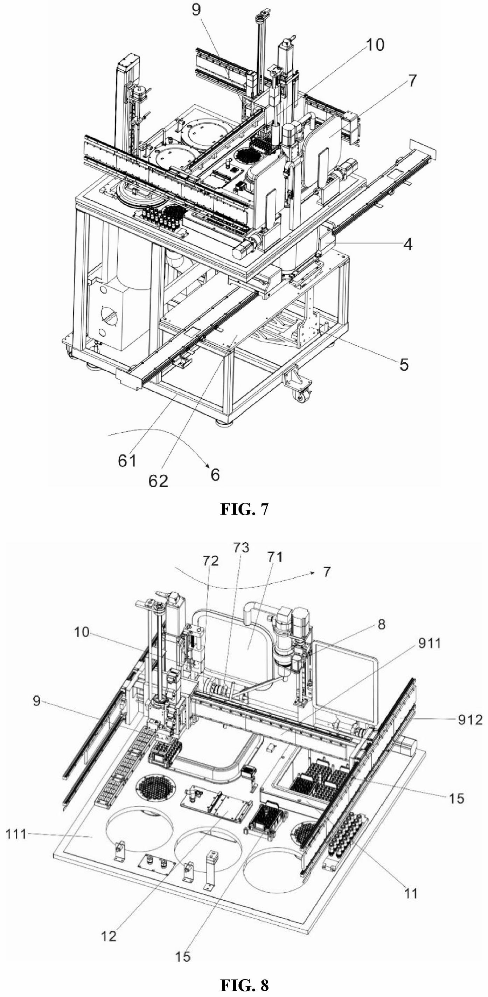

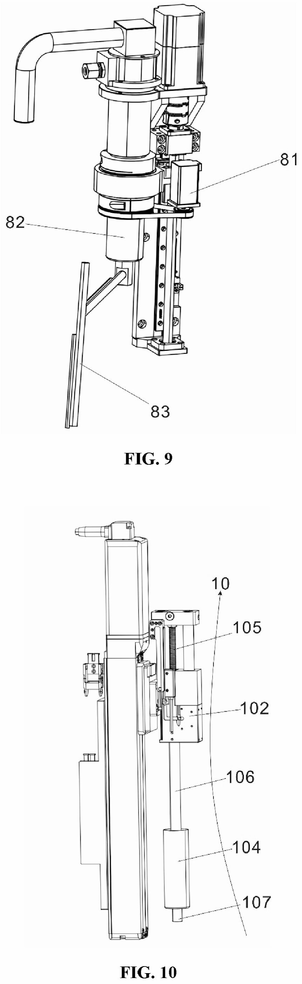

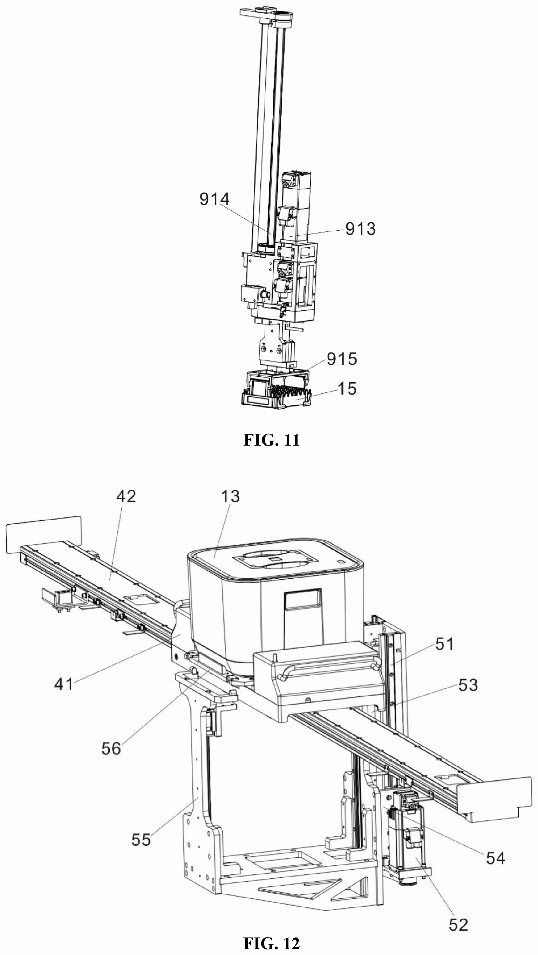

Referring to FIG. 7-FIG. 12, showing the second example of the present invention. On the basis of example 1, this example also comprises a multi-functional cooling system. The multi-functional cooling system comprises a transport assembly 4, a lifting assembly 5, a support rack assembly 6, a cover opening assembly 7, a rotatory liquid adding assembly 8, a grillage grabbing assembly 9, a pipette assembly 10, a sample temporary storage box 11, a barcode scanner 12 and a transfer tank 13. The transfer tank 13 can be transported by arranging the transport assembly, the transfer tank 13 can be lifted by arranging the lifting assembly 5 and docked with the cavity assembly 1, the upper cover of the transfer tank 13 can be opened by arranging the cover opening assembly 7, the liquid can be added into the transfer tank 13 and the sample temporary storage box 11 by arranging the rotatory liquid adding assembly 8, the single-tube biological sample or the whole-plate biological samples can be scanned and registered by arranging the barcode scanner 12, the single-tube biological sample or the whole-plate biological samples can be grabbed by arranging the grillage grabbing assembly 9 and the pipette assembly 10, the grillage grabbing assembly 9 and the pipette assembly 10 can grab the single-tube biological sample or the whole-plate biological samples in the transfer tank 13 to be temporarily put into the sample temporary storage box 11, and the grillage grabbing assembly 9 can place the biological grillages 15 on the grillage storage platform 230 or in the circulation barrel 315.

Specifically, the multi-functional cooling system comprises the transport assembly 4, the lifting assembly 5, the support rack assembly 6, the cover opening assembly 7, the rotatory liquid adding assembly 8, the grillage grabbing assembly 9, the pipette assembly 10, the sample temporary storage box 11, the barcode scanner 12 and the transfer tank 13, wherein
the support rack assembly 6 supports the transport assembly 4, the lifting assembly 5 and the cavity assembly 1, the transport assembly 4 transports the transfer tank 13, the lifting assembly 5 lifts up the transfer tank 13 and is docked with the cavity assembly 1, the cover opening assembly 7 opens an upper cover of the transfer tank 13, the rotatory liquid adding assembly 8 adds the liquid into the transfer tank 13, the pipette assembly 10 sucks redundant nitrogen, the grillage grabbing assembly 9 is used for grabbing the biological grillages 15, the sample temporary storage box 11 is used for placing the biological grillages 15, and the barcode scanner 12 scans the labels of the biological grillages 15.

Further, the cavity assembly 1 comprises an operating support plate 111 and an operating cavity housing 112, wherein the upper part of the operating support plate 111 is connected with the operating cavity housing 112, the operating support plate 111 and the operating cavity housing 112 form a closed operating cavity, and the operating cavity is internally provided with the cover opening assembly 7, the rotatory liquid adding assembly 8, the grillage grabbing assembly 9, the pipette assembly 10, the sample temporary storage box 11 and the barcode scanner 12.

Preferably, the operating cavity housing 112 is provided with an observation window by which the status of the inside of the operating cavity housing can be seen.

Further, the support rack assembly 6 comprises a support rack 61 and a support platform 62, wherein the upper end of the support rack 61 is fixedly connected with the operating support plate 111, and the side end of the support rack 61 is provided with the support platform 62.The support platform 62 is fixedly connected with the support rack 61.

Further, the transport assembly 4 comprises a transport vehicle 41 and a transport guide rail 42, wherein
the transport guide rail 42 is arranged on the upper part of the support platform 62, and the transport vehicle 41 is in sliding connection with the transport guide rail 42.

Preferably, the transport guide rail 42 is fixedly connected with the support platform 62.

Further, the lifting assembly 5 comprises a lifting guide rail 51, a lifting motor 52, a lifting lead screw 53, a lifting slider 54, a lifting U-shaped plate 55 and a lifting plate 56, wherein
the lifting guide rail 51 is vertically and fixedly connected with the support rack 61, the lifting slider 54 is in cooperative connection with the lifting lead screw 53, the upper end and lower end of the lifting guide rail 51 have bumps for supporting the lifting lead screw 53, the lifting motor 52 drives the lifting lead screw 53 to rotate through a belt wheel, the side surface of the lifting slider 54 is fixedly connected with the lifting U-shaped plate 55, the lifting plate 56 is arranged at the upper side of the middle of the transport vehicle 41, the transfer tank 13 is arranged on the upper end surface of the lifting plate 56, and the lifting U-shaped plate 55 makes the lifting plate 56 rise and fall.

Preferably, the upper part of the lifting U-shaped plate 55 is provided with a top claw, the lifting plate 56 is provided with a clip, and the top claw can be cooperated with the clip.

Preferably, the lifting motor 52 drives the lifting lead screw 53 to rotate through the belt wheel, the rotation of the lifting lead screw 53 drives the lifting slider 54 to vertically move along the lifting guide rail 51, the lifting slider 54 drives the top claw on the lifting U-shaped plate 55 to rise during the rising. After the top claw is cooperated with the clip on the lifting plate 56, the lifting plate 56 rises to drive the transfer tank 13 on the lifting plate 56 to rise, so that the transfer tank 13 is cooperated with the operating support plate 111.

Further, the cover opening assembly 7 comprises a door cover plate 71, a door cover motor 72 and a door cover spindle 73, wherein
the door cover motor 72 is connected with the door cover spindle 73, the door cover plate 71 is connected with the door cover spindle 73, the door cover spindle 73 performs supporting through a door cover support base, and the door cover support base is connected with the operating support plate 111.

Preferably, the upper cover of the transfer tank 13 can be arranged as being magnetic, the door cover plate 71 is also arranged as being magnetic, so as to open the upper cover of the transfer tank 13, or the upper cover of the transfer tank 13 can also be opened using other manners.

Preferably, the door cover motor 72 drives the door cover spindle 73 to drive the door cover plate 71 to open the upper cover of the transfer tank 13.The door cover plate 71 can also cover the notch accommodating the transfer tank 13 on the operating support plate 111 without the transfer tank 13, thereby maintaining the sealing of the cavity.

Further, the grillage grabbing assembly 9 comprises X-axis sliding rails 911, Y-axis sliding rails 912, a gripper motor 913, a gripper spindle 914 and a grillage gripper 915, wherein
the Y-axis sliding rails 912 are connected with the inner side of the operating cavity housing 112, the Y-axis sliding rails 912 are symmetrically arranged, two groups of symmetrical Y-axis sliding rails 912 are connected through the X-axis sliding rails 911, the X-axis sliding rails 911 are in sliding connection with the Y-axis sliding rails 912, the one sides of the X-axis sliding rails 911 are provided with the gripper motor 913, the gripper motor 913 is connected with the gripper spindle 914 through the belt wheel, and the lower part of the gripper spindle 914 is connected with the grillage gripper 915.

Preferably, the grillage gripper 915 can move on the X-axis sliding rail 911, and the X-axis sliding rail 911 can slide along the Y-axis sliding rail 912.The grillage gripper 915 can grab the biological grillages 15.

Preferably, the grillage grippers 915 can do up and down movement on the Z axis, the chains can drive a spindle rack and a motor rack to up and down move, and the motor rack can be in up and down sliding connection with the X-axis sliding rail, so that the grillage gripper 915 rises and falls; or by using other existing technologies, it is only needed to achieve the purpose of the grillage gripper 915 up and down rising and falling along the Z axis.

Preferably, the grillage gripper 915 can achieve movement along the directions of the XYZ axes. The grillage gripper 915 can grab the biological grillages 15.

Further, the pipette assembly 10 comprises an electric cylinder, a pipette motor 102, a liquid nitrogen cup 104, a pipette lead screw 105, a pipette 106 and a pipette tip 107, wherein
the electric cylinder is arranged at one side of the X-axis sliding rail 911 far away from the gripper motor 913, the side surface of the electric cylinder is provided with a motor guide rail, the pipette motor102 in sliding connection with the motor guide rail and the pipette lead screw 105 connected with the pipette motor 102, the lower part of the pipette motor 102 is connected with the pipette 106, the outer side of the pipette 106 is provided with the liquid nitrogen cup 104, and the lower end of the pipette 106 is connected with the pipette tip 107.

Preferably, the pipette motor 102 drives the pipette lead screw 105 to rotate, the pipette motor 102 rises and falls along the motor guide rail, the liquid nitrogen cup 104 can cool the pipette 106, and the pipette tip 107 can suck the single-tube sample; or the electric cylinder is used to drive the pipette motor 102 and the pipette 106 to rise and fall; or by using other existing technologies, the pipette 106 and the pipette tip 107 can do rising and falling movement.

Further, the sample temporary storage box 11 is arranged on the operating support plate 111, the biological grillages 15 are stored in the sample temporary storage box 11, the upper part of the sample temporary storage box 11 is provided with a second door cover plate, and a second door cover plate motor drives the second door cover plate to open or close the sample temporary storage box 11.

Preferably, the bottom of the sample temporary storage box 11 is connected with the liquid nitrogen valve 212 through a pipeline so as to add a liquid into the sample temporary storage box 11.The biological grillages 15 can be temporarily placed in the sample temporary storage box 11, and the second door cover plate can open and close the sample temporary storage box 11.

Further, the rotatory liquid adding assembly 8 comprises a rotatory liquid adding motor 81, a liquid adding barrel 82 and a liquid supplementing tube 83, wherein
the rotatory liquid adding motor 81 is connected with the liquid adding barrel 82 through the belt wheel, the liquid supplementing tube 83 is connected with the liquid adding barrel 82, the liquid adding barrel 82 performs support through the support shaft, and the support shaft is connected with the inner side surface of the operating cavity housing 112.

Preferably, the rotatory liquid adding motor 81 drives the liquid adding barrel 82 to rotate through the belt wheel; the liquid adding barrel 82 drives the liquid supplementing tube 83 to rotate; and by rotating, the liquid supplementing tube 83 can respectively add or supplement the liquid to the transfer tank 13 and the sample temporary storage box 11.

When in use, the transfer tank 13 is placed on the transport vehicle 41, the transport vehicle 41 moves above the lifting U-shaped plate 55 along the transfer guide rail 42, subsequently, the lifting motor 52 drives the lifting lead screw 53 to rotate through the belt wheel, the rotation of the lifting lead screw 53 drives the lifting slider 54 to vertically move along the lifting guide rail 51, the lifting slider 54 drives the top claw on the lifting U-shaped plate 55 to rise during the rising; and after the top claw is cooperated with the clip on the lifting plate 56, the lifting plate 56 rises so as to drive the transfer tank 13 on the lifting plate 56 to rise so that the transfer tank 13 is cooperated with the operating support plate 111, subsequently, the door cover motor 72 drives the door cover spindle 73 to drive the door cover plate 71 to open the upper cover of the transfer tank 13, the rotatory liquid adding motor 81 drives the liquid adding barrel 82 to rotate through the belt wheel, the liquid adding barrel 82 drives the liquid supplementing tube 83 to rotate, the liquid supplementing tube 83 adds or supplements the liquid to the transfer tank 13 or the sample temporary storage box 11 through the rotation, then the grillage gripper 915 moves through the XYZ axes under the instruction of the system to successively place the biological grillages 15 in the transfer tank 13 into the sample temporary storage box 11 for later use, or the single tube is grabbed by using the pipette tip 107 and then placed in the biological grillage 15, or the biological grillages 15 are grabbed by the grillage gripper 915 and then scanned, and then the scanned biological grillages 15 are placed in the circulation barrel 315 or in the grillage storage platform 230 for accessing and cooling.

In summary, through the transport assembly 4, the transfer tank 13 moves above the lifting assembly 5, the lifting assembly 5 drives the transfer tank 13 to rise to be docked with the cavity assembly 1; after docking, the cover opening assembly 7 can open the upper cover of the transfer tank 13, the biological grillages or the biological single-tube samples are grabbed by utilizing the grillage grabbing assembly 9 and the pipette assembly 10, so as to place the biological grillages or the biological single-tube samples in the cooling adjusting assembly 2 or the circulation cooling assembly 3, thereby achieving the cooling of the biological samples.

Importantly, it should be noted that the configurations and arrangements of the present application shown in multiple different exemplary embodiments are only illustrative. Although several embodiments are only described in detail in the disclosure here, persons who refer to the disclosure will readily appreciate that many modifications may be possible (for example, sizes, scales, structures, shapes and proportions of various elements, as well as parameters (for example, temperature, pressure, etc.), installation arrangement, use of materials, color, change in orientation, etc.) without substantially departing the novel teachings and advantages of the subject described in the present application. For example, an element shown as being integrally formed may be composed of multiple parts or elements, the positions of the elements may be reversed or otherwise varied, and the nature or number or position of discrete elements may be altered or varied. Therefore, all of such the modifications are intended to be included within the scope of the present invention. The order or sequence of any processes or method steps is changed or rearranged according to alternative embodiments. In claims, any clause of "device plus function" is intended to cover the structures described herein for implementing the functions, and is not only structurally equivalent but also an equivalent structure. Other replacements, modifications, changes and omissions can be made in the design, running state and arrangement of exemplary embodiments without departing from the scope of the present invention. Therefore, the present invention is not limited to specific embodiments, but extends to various modifications which still fall within the scope of the appended claims.

In addition, all features (i.e., those features irrelevant to the best mode presently contemplated for carrying out the present invention, or those features irrelevant to implementing the present invention) of actual embodiments can be not described in order to provide the brief description of exemplary embodiments.

It should be understood that a large amount of specific embodiments can be made in the development process of any actual embodiments such as any projects or design items. Such development efforts might be complex and time-consuming, but would not require undue experimentation for ordinary technicians having the benefit of this disclosure, which would be a routine work of design, manufacture and production.

## Claims

1. A biological sample cooling device, comprising a cavity assembly (1), a cooling adjusting assembly (2), a circulation cooling assembly (3) and biological grillages (15), wherein
the cavity assembly (1) is provided with the cooling adjusting assembly (2) and the circulation cooling assembly (3), the circulation cooling assembly (3) is arranged at the side of the cooling adjusting assembly (2), and the cooling adjusting assembly (2) is configured to individually perform gradient cooling on the biological grillages (15); multiple groups of the biological grillages (15) are accommodated inside the circulation cooling assembly (3), and the circulation cooling assembly (3) is configured to perform adjustable temperature control on the multiple groups of the biological grillages (15) inside the circulation cooling assembly (3);
**characterized in that** the circulation cooling assembly (3) comprises a circulation barrel member (31), an air suction member (33) and a heating member (35), wherein
the circulation barrel member (31) is connected and fixed with the cavity assembly (1), the lower end of the circulation barrel member (31) is configured to communicate with the heating member (35) through a pipeline, the heating member (35) is configured to communicate with the air suction member (33), and the air suction member (33) is connected with the cavity assembly (1), such that a gas inside the cavity assembly (1) is sucked by the air suction member (33) and heated by the heating member (35), then the heated gas and liquid nitrogen are mixed in the circulation barrel member (31) so that the temperature of liquid nitrogen increases, thereby avoiding quick cooling.

2. The biological sample cooling device according to claim 1, **characterized in that** the cooling adjusting assembly (2) comprises a cooling barrel member (21) and a lifting and accessing member (22), wherein
the upper part of the cooling barrel member (21) is provided with the lifting and accessing member (22), the lifting and accessing member (22) is configured to drive the biological grillages (15) to enter the cooling barrel member (21), the cooling barrel member (21) is internally provided with liquid nitrogen, the cooling barrel member (21) is configured to control the temperature of the liquid nitrogen, and the lifting and accessing member (22) is configured to detect the temperatures of the biological grillages (15) and the environment temperature of the biological grillages (15).

3. The biological sample cooling device according to claim 2, **characterized in that** the cooling barrel member (21) comprises a cooling vacuum barrel (211), a liquid nitrogen valve (212) and a liquid adding pipeline (213), wherein
the cooling vacuum barrel (211) is connected and fixed with the cavity assembly (1), and the liquid nitrogen valve (212) is configured to control the liquid adding pipeline (213) to place the liquid nitrogen into the cooling vacuum barrel (211).

4. The biological sample cooling device according to claim 3, **characterized in that** the lifting and accessing member (22) comprises a support plate (221), a support sliding plate (222), a guide rail support plate (223), a lead screw (224), a landing slider (225), a fixed block (226), a buffer spring (227), a temperature sensor (228), a sample temperature detecting tube (229), a grillage storage platform (230), a first motor (231) and a slider guide rail (232), wherein
the side surface of the support plate (221) is provided with the support sliding plate (222), the side surface of the support sliding plate (222) is connected with the guide rail support plate (223), the side surface of the upper end of the guide rail support plate (223) is connected with a first motor support platform, the first motor support platform is equipped with the first motor (231), the first motor (231) is vertically connected with the lead screw (224) through a transmission shaft, the lead screw (224) is connected with the fixed block (226) and the landing slider (225), the side surface of the guide rail support plate (223) is fixedly provided with the slider guide rail (232), the landing slider (225) and the fixed block (226) are in sliding connection with the slider guide rail (232), the lower end of the fixed block (226) is connected with the buffer spring (227), the lower end of the buffer spring (227) is connected with the temperature sensor (228), the lower end of the temperature sensor (228) is provided with the sample temperature detecting tube (229), the lower end of the guide rail support plate (223) is **connected** with the grillage storage platform (230), and the grillage storage platform (230) is configured to be used for placing the biological grillages (15); and the side surface of the support sliding plate (222) is connected with chains, and the support sliding plate (222) is in up and down sliding connection with the support plate (221).

5. The biological sample cooling device according to claim 1, **characterized in that** the air suction member (33) comprises an air blower (331), a mesh plate (332) and an air suction tube (333), wherein
the upper part of the air blower (331) is connected with the mesh plate (332), and the air blower (331) is configured to communicate with the air suction tube (333).

6. The biological sample cooling device according to claim 5, **characterized in that** the heating member (35) comprises a heating module (351) and an air inlet tube (352), wherein
the heating module (351) is configured to communicate with the air suction tube (333), and the heating module (351) is configured to communicate with the air inlet tube (352).

7. The biological sample cooling device according to claim 6, **characterized in that** the circulation barrel member (31) comprises a docking cavity (311), nitrogen injection tubes (312), gas ejector tubes (313), a guided cooling dish (314) and a circulation barrel (315), wherein
the air inlet tube (352) is configured to communicate with the docking cavity (311), the docking cavity (311) is arranged inside the circulation barrel (315), the nitrogen injection tubes (312) are arranged around the docking cavity (311) and configured to communicate with the docking cavity (311), the gas ejector tubes (313) are arranged on the upper parts of the nitrogen injection tubes (312), the gas ejector tubes (313) are configured to communicate with the docking cavity (311) through pipelines, multiple groups of the gas ejector tubes (313) and the nitrogen injection tubes (312) are circumferentially arranged, the gas ejector tubes (313) are upwardly provided with round holes, the upper parts of the gas ejector tubes (313) are provided with the guided cooling dish (314), the guided cooling dish (314) is provided with meshed-shaped holes, the upper part of the guided cooling dish (314) is provided with grillage columns, and the biological grillages (15) are placed inside the circulation barrel (315) through the grillage columns; and multiple groups of the biological grillages (15) are arranged inside the circulation barrel (315).

8. The biological sample cooling device according to claim 7, **characterized in that** the nitrogen injection tube (312) is configured to communicate with the liquid adding pipeline (213).

9. A multi-functional cooling system, comprising the biological sample cooling device according to any one of claims 1-8, and also comprising a transport assembly (4), a lifting assembly (5), a support rack assembly (6), a cover opening assembly (7), a rotatory liquid adding assembly (8), a grillage grabbing assembly (9), a pipette assembly (10), a sample temporary storage box (11), a barcode scanner (12) and a transfer tank (13), wherein
the support rack assembly (6) is configured to support the transport assembly (4), the lifting assembly (5) and the cavity assembly (1), the transport assembly (4) is configured to transport the transfer tank (13), the lifting assembly (5) is configured to lift up the transfer tank (13) and to be docked with the cavity assembly (1), the cover opening assembly (7) is configured to open an upper cover of the transfer tank (13), the rotatory liquid adding assembly (8) is configured to add the liquid into the transfer tank (13), the pipette assembly (10) is configured to suck redundant nitrogen, the grillage grabbing assembly (9) is configured to be used for grabbing the biological grillages (15), the sample temporary storage box (11) is configured to be used for placing the biological grillages (15), and the barcode scanner (12) is configured to scan the labels of the biological grillages (15).

10. The multi-functional cooling system according to claim 9, **characterized in that** the cavity assembly (1) comprises an operating support plate (111) and an operating cavity housing (112), wherein the upper part of the operating support plate (111) is connected with the operating cavity housing (112), the operating support plate (111) and the operating cavity housing (112) form an operating cavity, and the operating cavity is internally provided with the cover opening assembly (7), the rotatory liquid adding assembly (8), the grillage grabbing assembly (9), the pipette assembly (10), the sample temporary storage box (11) and the barcode scanner (12).

11. The multi-functional cooling system according to claim 10, **characterized in that** the support rack assembly (6) comprises a support rack (61) and a support platform (62), wherein the upper end of the support rack (61) is connected with the operating support plate (111), and the side end of the support rack (61) is provided with the support platform (62).

12. The multi-functional cooling system according to claim 11, **characterized in that** the transport assembly (4) comprises a transport vehicle (41) and a transport guide rail (42), wherein
the transport guide rail (42) is arranged on the upper part of the support platform (62), and the transport vehicle (41) is in sliding connection with the transport guide rail (42).

13. The multi-functional cooling system according to claim 12, **characterized in that** the lifting assembly (5) comprises a lifting guide rail (51), a lifting motor (52), a lifting lead screw (53), a lifting slider (54), a lifting U-shaped plate (55) and a lifting plate (56), wherein
the lifting guide rail (51) is vertically and fixedly connected with the support rack (61), the lifting slider (54) is in cooperative connection with the lifting lead screw (53), the upper end and lower end of the lifting guide rail (51) have bumps for supporting the lifting lead screw (53), the lifting motor (52) is configured to drive the lifting lead screw (53) to rotate through a belt wheel, the side surface of the lifting slider (54) is fixedly connected with the lifting U-shaped plate (55), the lifting plate (56) is arranged at the upper side of the middle of the transport vehicle (41), the transfer tank (13) is arranged on the upper end surface of the lifting plate (56), and the lifting plate (56) is configured to rise and fall through the lifting U-shaped plate (55).

14. The multi-functional cooling system according to claim 13, **characterized in that** the cover opening assembly (7) comprises a door cover plate (71), a door cover motor (72) and a door cover spindle (73), wherein
the door cover motor (72) is connected with the door cover spindle (73), the door cover plate (71) is connected with the door cover spindle (73), the door cover spindle (73) is configured to perform supporting through a door cover support base, and the door cover support base is connected with the operating support plate (111).

15. The multi-functional cooling system according to claim 14, **characterized in that** the grillage grabbing assembly (9) comprises X-axis sliding rails (911), Y-axis sliding rails (912), a gripper motor (913), a gripper spindle (914) and grillage grippers (915), wherein
the Y-axis sliding rails (912) are connected with the inner side of the operating cavity housing (112), the Y-axis sliding rails (912) are symmetrically arranged, two groups of symmetrical Y-axis sliding rails (912) are connected through the X-axis sliding rails (911), the X-axis sliding rails (911) are in sliding connection with the Y-axis sliding rails (912), one sides of the X-axis sliding rails (911) are provided with the gripper motor (913), the gripper motor (913) is connected with the gripper spindle (914) through the belt wheel, and the lower part of the gripper spindle (914) is connected with the grillage grippers (915).

16. The multi-functional cooling system according to claim 15, **characterized in that** the pipette assembly (10) comprises an electric cylinder, a pipette motor (102), a liquid nitrogen cup (104), a pipette lead screw (105), a pipette (106) and a pipette tip (107), wherein
the electric cylinder is arranged at one sides of the X-axis sliding rails (911) far away from the gripper motor (913), the side surface of the electric cylinder is provided with a motor guide rail, the pipette motor (102) in sliding connection with the motor guide rail and the pipette lead screw (105) connected with the pipette motor (102), the lower end of the pipette motor (102) is connected with the pipette (106), the outer side of the pipette (106) is provided with the liquid nitrogen cup (104), and the lower end of the pipette (106) is connected with the pipette tip (107).

17. The multi-functional cooling system according to claim 16, **characterized in that** the sample temporary storage box (11) is arranged on the operating support plate (111), the biological grillages (15) are stored in the sample temporary storage box (11), the upper part of the sample temporary storage box (11) is provided with a second door cover plate, and a second door cover plate motor is configured to drive the second door cover plate to open or close the sample temporary storage box (11).

18. The multi-functional cooling system according to claim 17, **characterized in that** the rotatory liquid adding assembly (8) comprises a rotatory liquid adding motor (81), a liquid adding barrel (82) and a liquid supplementing tube (83), wherein
the rotatory liquid adding motor (81) is connected with the liquid adding barrel (82) through the belt wheel, the liquid supplementing tube (83) is connected with the liquid adding barrel (82), the liquid adding barrel (82) is configured to perform supporting through the support shaft, and the support shaft is connected with the inner side surface of the operating cavity housing (112).

## Patentansprüche

1. Biologische Probenkühlvorrichtung, umfassend eine Hohlraumbaugruppe (1), eine Kühlverstellbaugruppe (2), eine Umlaufkühlbaugruppe (3) und biologische Probengestelle (15), wobei
die Hohlraumbaugruppe (1) mit der Kühlverstellbaugruppe (2) und der Umlaufkühlbaugruppe (3) versehen ist, wobei die Umlaufkühlbaugruppe (3) an einer Seite der Kühlverstellbaugruppe (2) angeordnet ist, und wobei die Kühlverstellbaugruppe (2) so konfiguriert ist, eine Gradientenkühlung der biologischen Probengestelle (15) einzeln durchzuführen; wobei mehrere Gruppen biologischer Probengestelle (15) im Inneren der Umlaufkühlbaugruppe (3) aufgenommen sind, und wobei die Umlaufkühlbaugruppe (3) so konfiguriert ist, eine einstellbare Temperaturregelung der mehreren Gruppen der biologischen Probengestelle (15) im Inneren der Umlaufkühlbaugruppe (3) durchzuführen;
**dadurch gekennzeichnet, dass** die Umlaufkühlbaugruppe (3) ein Umlaufzylinderelement (31), ein Luftansaugelement (33) und ein Heizelement (35) umfasst, wobei
das Umlaufzylinderelement (31) mit der Hohlraumbaugruppe (1) verbunden und daran befestigt ist, wobei ein unteres Ende des Umlaufzylinderelements (31) so konfiguriert ist, über eine Leitung mit dem Heizelement (35) in Verbindung zu stehen, wobei das Heizelement (35) so konfiguriert ist, mit dem Luftansaugelement (33) in Verbindung zu stehen, und wobei das Luftansaugelement (33) mit der Hohlraumbaugruppe (1) verbunden ist, derart, dass ein Gas im Inneren der Hohlraumbaugruppe (1) von dem Luftansaugelement (33) angesaugt und von dem Heizelement (35) erhitzt wird, wonach das erwärmte Gas und flüssiger Stickstoff in dem Umlaufzylinderelement (31) gemischt werden, so dass sich die Temperatur des flüssigen Stickstoffs erhöht, wodurch ein übermäßig schnelles Abkühlen vermieden wird.

2. Biologische Probenkühlvorrichtung nach Anspruch 1, **dadurch gekennzeichnet, dass** die Kühlverstellbaugruppe (2) ein Kühlzylinderelement (21) und ein Hub- und Zugriffselement (22) umfasst, wobei
ein oberer Teil des Kühlzylinderelements (21) mit dem Hub- und Zugriffselement (22) versehen ist, wobei das Hub- und Zugriffselement (22) so konfiguriert ist, die biologischen Probengestelle (15) zum Eintritt in das Kühlzylinderelement (21) anzutreiben, wobei das Kühlzylinderelement (21) im Inneren mit flüssigem Stickstoff versehen ist, wobei das Kühlzylinderelement (21) so konfiguriert ist, die Temperatur des flüssigen Stickstoffs zu regeln, und wobei das Hub- und Zugriffselement (22) so konfiguriert ist, die Temperaturen der biologischen Probengestelle (15) und die Umgebungstemperatur der biologischen Probengestelle (15) zu erfassen.

3. Biologische Probenkühlvorrichtung nach Anspruch 2, **dadurch gekennzeichnet, dass** das Kühlzylinderelement (21) einen Kühlvakuumzylinder (211), ein Flüssigstickstoffventil (212) und eine Flüssigkeitszugabeleitung (213) umfasst, wobei
der Kühlvakuumzylinder (211) mit der Hohlraumbaugruppe (1) verbunden und daran befestigt ist, wobei das Flüssigstickstoffventil (212) so konfiguriert ist, die Flüssigkeitszugabeleitung (213) derart zu regeln, dass der flüssige Stickstoff in den Kühlvakuumzylinder (211) eingebracht wird.

4. Biologische Probenkühlvorrichtung nach Anspruch 3, **dadurch gekennzeichnet, dass** das Hub- und Zugriffselement (22) eine Stützplatte (221), eine Stützschiebeplatte (222), eine Führungsschienenstützplatte (223), eine Gewindespindel (224), einen Hubschlitten (225), einen Befestigungsblock (226), eine Pufferfeder (227), einen Temperatursensor (228), ein Probentemperaturfühlerrohr (229), eine Gestelllagerplattform (230), einen ersten Motor (231) und eine Schlittenführungsschiene (232) umfasst, wobei
eine Seitenfläche der Stützplatte (221) mit der Stützschiebeplatte (222) versehen ist, wobei eine Seitenfläche der Stützschiebeplatte (222) mit der Führungsschienenstützplatte (223) verbunden ist, wobei eine Seitenfläche eines oberen Endes der Führungsschienenstützplatte (223) mit einer ersten Motorträgerplattform verbunden ist, wobei die erste Motorträgerplattform mit dem ersten Motor (231) ausgestattet ist, wobei der erste Motor (231) über eine Getriebewelle vertikal mit der Gewindespindel (224) verbunden ist, wobei die Gewindespindel (224) mit dem Befestigungsblock (226) und dem Hubschlitten (225) verbunden ist, wobei die Seitenfläche der Führungsschienenstützplatte (223) fest mit der Schlittenführungsschiene (232) versehen ist, wobei der Hubschlitten (225) und der Befestigungsblock (226) mit der Schlittenführungsschiene (232) verschiebbar verbunden sind, wobei ein unteres Ende des Befestigungsblocks (226) mit der Pufferfeder (227) verbunden ist, wobei ein unteres Ende der Pufferfeder (227) mit dem Temperatursensor (228) verbunden ist, wobei ein unteres Ende des Temperatursensors (228) mit dem Probentemperaturfühlerrohr (229) versehen ist, wobei ein unteres Ende der Führungsschienenstützplatte (223) mit der Gestelllagerplattform (230) verbunden ist, und wobei die Gestelllagerplattform (230) so konfiguriert ist, zum Platzieren der biologischen Probengestelle (15); und wobei die Seitenfläche der Stützschiebeplatte (222) mit Ketten verbunden ist, und wobei die Stützschiebeplatte (222) mit der Stützplatte (221) auf und ab verschiebbar verbunden ist.

5. Biologische Probenkühlvorrichtung nach Anspruch 1, **dadurch gekennzeichnet, dass** das Luftansaugelement (33) ein Gebläse (331), eine Gitterplatte (332) und ein Luftansaugrohr (333) umfasst, wobei
ein oberer Teil des Gebläses (331) mit der Gitterplatte (332) verbunden ist, und wobei das Gebläse (331) so konfiguriert ist, mit dem Luftansaugrohr (333) in Verbindung zu stehen.

6. Biologische Probenkühlvorrichtung nach Anspruch 5, **dadurch gekennzeichnet, dass** das Heizelement (35) ein Heizmodul (351) und ein Lufteinlassrohr (352) umfasst, wobei
das Heizmodul (351) so konfiguriert ist, mit dem Luftansaugrohr (333) in Verbindung zu stehen, und das Heizmodul (351) so konfiguriert ist, mit dem Lufteinlassrohr (352) in Verbindung zu stehen.

7. Biologische Probenkühlvorrichtung nach Anspruch 6, **dadurch gekennzeichnet, dass** das Umlaufzylinderelement (31) eine Andockkammer (311), Stickstoffeinspritzrohre (312), Gasausstoßrohre (313), eine geführte Kühlschale (314) und einen Umlaufzylinder (315) umfasst, wobei
das Lufteinlassrohr (352) so konfiguriert ist, mit der Andockkammer (311) in Verbindung zu stehen, wobei die Andockkammer (311) im Inneren des Umlaufzylinders (315) angeordnet ist, wobei die Stickstoffeinspritzrohre (312) um die Andockkammer (311) herum angeordnet und so konfiguriert sind, mit der Andockkammer (311) in Verbindung zu stehen, wobei die Gasausstoßrohre (313) an oberen Teilen der Stickstoffeinspritzrohre (312) angeordnet sind, wobei die Gasausstoßrohre (313) so konfiguriert sind, über Leitungen mit der Andockkammer (311) in Verbindung zu stehen, wobei mehrere Gruppen der Gasausstoßrohre (313) und Stickstoffeinspritzrohre (312) umfänglich angeordnet sind, wobei die Gasausstoßrohre (313) nach oben mit Rundlöchern versehen sind, wobei obere Teile der Gasausstoßrohre (313) mit der geführten Kühlschale (314) versehen sind, wobei die geführte Kühlschale (314) mit gitterförmigen Löchern versehen ist, wobei ein oberer Teil der geführten Kühlschale (314) mit Gestellsäulen versehen ist, und wobei die biologischen Probengestelle (15) durch die Gestellsäulen im Inneren des Umlaufzylinders (315) platziert sind; und wobei die mehreren Gruppen der biologischen Probengestelle (15) im Inneren des Umlaufzylinders (315) angeordnet sind.

8. Biologische Probenkühlvorrichtung nach Anspruch 7, **dadurch gekennzeichnet, dass** das Stickstoffeinspritzrohr (312) so konfiguriert ist, mit der Flüssigkeitszugabeleitung (213) in Verbindung zu stehen.

9. Multifunktionales Kühlsystem, umfassend die biologische Probenkühlvorrichtung nach einem der Ansprüche 1 bis 8, und ferner umfassend eine Transportbaugruppe (4), eine Hubbaugruppe (5), eine Tragrahmenbaugruppe (6), eine Deckelöffnungsbaugruppe (7), eine Rotationsflüssigkeitszugabebaugruppe (8), eine Gestellgreifbaugruppe (9), eine Pipettenbaugruppe (10), eine Probenzwischenlagerbox (11), einen Barcodescanner (12) und einen Transfertank (13), wobei
die Tragrahmenbaugruppe (6) so konfiguriert ist, die Transportbaugruppe (4), die Hubbaugruppe (5) und die Hohlraumbaugruppe (1) zu tragen, wobei die Transportbaugruppe (4) so konfiguriert ist, den Transfertank (13) zu transportieren, wobei die Hubbaugruppe (5) so konfiguriert ist, den Transfertank (13) anzuheben und mit der Hohlraumbaugruppe (1) verbunden zu werden, wobei die Deckelöffnungsbaugruppe (7) so konfiguriert ist, einen Oberdeckel des Transfertanks (13) zu öffnen, wobei die Rotationsflüssigkeitszugabebaugruppe (8) so konfiguriert ist, die Flüssigkeit in den Transfertank (13) einzubringen, wobei die Pipettenbaugruppe (10) so konfiguriert ist, überschüssigen Stickstoff abzusaugen, wobei die Gestellgreifbaugruppe (9) so konfiguriert ist, zum Greifen der biologischen Probengestelle (15) verwendet zu werden, wobei die Probenzwischenlagerbox (11) so konfiguriert ist, zum Platzieren der biologischen Probengestelle (15) verwendet zu werden, und wobei der Barcodescanner (12) so konfiguriert ist, Etiketten der biologischen Probengestelle (15) zu scannen.

10. Multifunktionales Kühlsystem nach Anspruch 9, **dadurch gekennzeichnet, dass** die Hohlraumbaugruppe (1) eine Betriebsstützplatte (111) und ein Betriebshohlraumgehäuse (112) umfasst,
wobei ein oberer Teil der Betriebsstützplatte (111) mit dem Betriebshohlraumgehäuse (112) verbunden ist, wobei die Betriebsstützplatte (111) und das Betriebshohlraumgehäuse (112) einen Betriebshohlraum bilden, und wobei der Betriebshohlraum im Inneren mit der Deckelöffnungsbaugruppe (7), der Rotationsflüssigkeitszugabebaugruppe (8), der Gestellgreifbaugruppe (9), der Pipettenbaugruppe (10), der Probenzwischenlagerbox (11) und dem Barcodescanner (12) versehen ist.

11. Multifunktionales Kühlsystem nach Anspruch 10, **dadurch gekennzeichnet, dass** die Tragrahmenbaugruppe (6) einen Tragrahmen (61) und eine Stützplattform (62) umfasst, wobei ein oberes Ende des Tragrahmens (61) mit der Betriebsstützplatte (111) verbunden ist, und wobei ein Seitenende des Tragrahmens (61) mit der Stützplattform (62) versehen ist.

12. Multifunktionales Kühlsystem nach Anspruch 11, **dadurch gekennzeichnet, dass** die Transportbaugruppe (4) ein Transportfahrzeug (41) und eine Transportführungsschiene (42) umfasst, wobei
die Transportführungsschiene (42) an einem oberen Teil der Stützplattform (62) angeordnet ist, und wobei das Transportfahrzeug (41) mit der Transportführungsschiene (42) verschiebbar verbunden ist.

13. Multifunktionales Kühlsystem nach Anspruch 12, **dadurch gekennzeichnet, dass** die Hubbaugruppe (5) eine Hubführungsschiene (51), einen Hubmotor (52), eine Hubgewindespindel (53), einen Hubschlitten (54), eine U-förmige Hubplatte (55) und eine Hubplatte (56) umfasst, wobei
die Hubführungsschiene (51) vertikal und fest mit dem Tragrahmen (61) verbunden ist, wobei der Hubschlitten (54) mit der Hubgewindespindel (53) zusammenwirkend verbunden ist, wobei ein oberes Ende und ein unteres Ende der Hubführungsschiene (51) Erhebungen zum Tragen der Hubgewindespindel (53) aufweisen, wobei der Hubmotor (52) so konfiguriert ist, die Hubgewindespindel (53) über ein Riemenrad zum Drehen anzutreiben, wobei eine Seitenfläche des Hubschlittens (54) fest mit der U-förmigen Hubplatte (55) verbunden ist, wobei die Hubplatte (56) an einer oberen Seite der Mitte des Transportfahrzeugs (41) angeordnet ist, wobei der Transfertank (13) an einer oberen Endfläche der Hubplatte (56) angeordnet ist, und wobei die Hubplatte (56) so konfiguriert ist, durch die U-förmige Hubplatte (55) anzuheben und abzusenken.

14. Multifunktionales Kühlsystem nach Anspruch 13, **dadurch gekennzeichnet, dass** die Deckelöffnungsbaugruppe (7) eine Türdeckelplatte (71), einen Türdeckelmotor (72) und eine Türdeckelspindel (73) umfasst, wobei
der Türdeckelmotor (72) mit der Türdeckelspindel (73) verbunden ist, wobei die Türdeckelplatte (71) mit der Türdeckelspindel (73) verbunden ist, wobei die Türdeckelspindel (73) so konfiguriert ist, eine Abstützung über einen Türdeckelstützsockel durchzuführen, und wobei der Türdeckelstützsockel mit der Betriebsstützplatte (111) verbunden ist.

15. Multifunktionales Kühlsystem nach Anspruch 14, **dadurch gekennzeichnet, dass** die Gestellgreifbaugruppe (9) X-Achsen-Schiebeschienen (911), Y-Achsen-Schiebeschienen (912), einen Greifermotor (913), eine Greiferspindel (914) und Gestellgreifer (915) umfasst, wobei
die Y-Achsen-Schiebeschienen (912) mit der Innenseite des Betriebshohlraumgehäuses (112) verbunden sind, wobei die Y-Achsen-Schiebeschienen (912) symmetrisch angeordnet sind, wobei zwei Gruppen symmetrischer Y-Achsen-Schiebeschienen (912) durch die X-Achsen-Schiebeschienen (911) verbunden sind, wobei die X-Achsen-Schiebeschienen (911) mit den Y-Achsen-Schiebeschienen (912) verschiebbar verbunden sind, wobei eine Seite der X-Achsen-Schiebeschienen (911) mit dem Greifermotor (913) versehen ist, wobei der Greifermotor (913) über das Riemenrad mit der Greiferspindel (914) verbunden ist, und wobei ein unterer Teil der Greiferspindel (914) mit den Gestellgreifern (915) verbunden ist.

16. Multifunktionales Kühlsystem nach Anspruch 15, **dadurch gekennzeichnet, dass** die Pipettenbaugruppe (10) einen Elektrozylinder, einen Pipettenmotor (102), einen Flüssigstickstoffbecher (104), eine Pipettengewindespindel (105), eine Pipette (106) und eine Pipettenspitze (107) umfasst, wobei
der Elektrozylinder an einer Seite der X-Achsen-Schiebeschienen (911), die von dem Greifermotor (913) abgewandt ist, angeordnet ist, wobei eine Seitenfläche des Elektrozylinders mit einer Motorführungsschiene versehen ist, wobei der Pipettenmotor (102) mit der Motorführungsschiene verschiebbar verbunden ist und die Pipettengewindespindel (105) mit dem Pipettenmotor (102) verbunden ist, wobei ein unteres Ende des Pipettenmotors (102) mit der Pipette (106) verbunden ist, wobei eine Außenseite der Pipette (106) mit dem Flüssigstickstoffbecher (104) versehen ist, und wobei ein unteres Ende der Pipette (106) mit der Pipettenspitze (107) verbunden ist.

17. Multifunktionales Kühlsystem nach Anspruch 16, **dadurch gekennzeichnet, dass** die Probenzwischenlagerbox (11) auf der Betriebsstützplatte (111) angeordnet ist, wobei die biologischen Probengestelle (15) in der Probenzwischenlagerbox (11) gelagert sind, wobei ein oberer Teil der Probenzwischenlagerbox (11) mit einer zweiten Türdeckelplatte versehen ist, und wobei ein zweiter Türdeckelplattenmotor so konfiguriert ist, die zweite Türdeckelplatte zum Öffnen oder Schließen der Probenzwischenlagerbox (11) anzutreiben.

18. Multifunktionales Kühlsystem nach Anspruch 17, **dadurch gekennzeichnet, dass** die Rotationsflüssigkeitszugabebaugruppe (8) einen Rotationsflüssigkeitszugabemotor (81), einen Flüssigkeitszugabezylinder (82) und ein Flüssigkeitsergänzungsrohr (83) umfasst, wobei
der Rotationsflüssigkeitszugabemotor (81) über das Riemenrad mit dem Flüssigkeitszugabezylinder (82) verbunden ist, wobei das Flüssigkeitsergänzungsrohr (83) mit dem Flüssigkeitszugabezylinder (82) verbunden ist, wobei der Flüssigkeitszugabezylinder (82) so konfiguriert ist, eine Abstützung über eine Abstützwelle durchzuführen, und wobei die Abstützwelle mit einer inneren Seitenfläche des Betriebshohlraumgehäuses (112) verbunden ist.

## Revendications

1. Un dispositif de refroidissement d'échantillons biologiques, comprenant un ensemble de cavité (1), un ensemble de réglage du refroidissement (2), un ensemble de refroidissement par circulation (3) et des grilles biologiques (15), dans lequel
l'ensemble de cavité (1) est pourvu de l'ensemble de réglage du refroidissement (2) et de l'ensemble de refroidissement par circulation (3), l'ensemble de refroidissement par circulation (3) est disposé sur le côté de l'ensemble de réglage du refroidissement (2), et l'ensemble de réglage du refroidissement (2) est configuré pour effectuer individuellement un refroidissement selon un gradient sur les grilles biologiques (15) ; plusieurs groupes de grilles biologiques (15) sont logés à l'intérieur de l'ensemble de refroidissement par circulation (3), et l'ensemble de refroidissement par circulation (3) est configuré pour effectuer un contrôle réglable de la température de plusieurs groupes de grilles biologiques (15) à l'intérieur de l'ensemble de refroidissement par circulation (3) ;
**caractérisé en ce que** l'ensemble de refroidissement par circulation (3) comprend un corps cylindrique de circulation (31), un élément d'aspiration d'air (33) et un élément chauffant (35), dans lequel
le corps cylindrique de circulation (31) est raccordé et fixé à l'ensemble de cavité (1), l'extrémité inférieure du corps cylindrique de circulation (31) est configurée pour être en communication avec l'élément chauffant (35) par l'intermédiaire d'une conduite, l'élément chauffant (35) est configuré pour être en communication avec l'élément d'aspiration d'air (33), et l'élément d'aspiration d'air (33) est raccordé à l'ensemble de cavité (1), de sorte qu'un gaz à l'intérieur de l'ensemble de cavité (1) est aspiré par l'élément d'aspiration d'air (33) et chauffé par l'élément chauffant (35), puis le gaz chauffé et l'azote liquide sont mélangés dans le corps cylindrique de circulation (31) de sorte que la température de l'azote liquide augmente, évitant ainsi un refroidissement rapide.

2. Le dispositif de refroidissement d'échantillons biologiques selon la revendication 1, **caractérisé en ce que** l'ensemble de réglage du refroidissement (2) comprend un corps cylindrique de refroidissement (21) et un élément de levage et d'accès (22), dans lequel
la partie supérieure du corps cylindrique de refroidissement (21) est pourvue de l'élément de levage et d'accès (22), l'élément de levage et d'accès (22) est configuré pour entraîner les grilles biologiques (15) à l'intérieur du corps cylindrique de refroidissement (21), le corps cylindrique de refroidissement (21) contient intérieurement de l'azote liquide, le corps cylindrique de refroidissement (21) est configuré pour réguler la température de l'azote liquide, et l'élément de levage et d'accès (22) est configuré pour détecter les températures des grilles biologiques (15) et la température ambiante des grilles biologiques (15).

3. Le dispositif de refroidissement d'échantillons biologiques selon la revendication 2, **caractérisé en ce que** le corps cylindrique de refroidissement (21) comprend une cuve de refroidissement sous vide (211), une vanne d'azote liquide (212) et une conduite d'alimentation en liquide (213), dans lequel
la cuve de refroidissement sous vide (211) est reliée et fixée à l'ensemble de cavité (1), et la vanne d'azote liquide (212) est configurée pour commander la conduite d'alimentation en liquide (213) afin d'introduire l'azote liquide dans la cuve de refroidissement sous vide (211).

4. Le dispositif de refroidissement d'échantillons biologiques selon la revendication 3, **caractérisé en ce que** l'élément de levage et d'accès (22) comprend une plaque de support (221), une plaque coulissante de support (222), une plaque de support de rail de guidage (223), une vis-mère (224), un coulisseau de descente (225), un bloc fixe (226), un ressort d'amortissement (227), un capteur de température (228), un tube de détection de la température de l'échantillon (229), une plateforme de stockage des grilles (230), un premier moteur (231) et un rail de guidage du coulisseau (232), dans lequel
la surface latérale de la plaque de support (221) est pourvue de la plaque coulissante de support (222), la surface latérale de la plaque coulissante de support (222) est reliée à la plaque de support de rail de guidage (223), la surface latérale de l'extrémité supérieure de la plaque de support de rail de guidage (223) est reliée à une plateforme de support du premier moteur, la plateforme de support du premier moteur est équipée du premier moteur (231), le premier moteur (231) est relié verticalement à la vis-mère (224) par l'intermédiaire d'un arbre de transmission, la vis-mère (224) est reliée au bloc fixe (226) et au coulisseau de descente (225), la surface latérale de la plaque de support de rail de guidage (223) est pourvue de manière fixe du rail de guidage du coulisseau (232), le coulisseau de descente (225) et le bloc fixe (226) sont reliés de manière coulissante au rail de guidage du coulisseau (232), l'extrémité inférieure du bloc fixe (226) est reliée au ressort d'amortissement (227), l'extrémité inférieure du ressort d'amortissement (227) est reliée au capteur de température (228), l'extrémité inférieure du capteur de température (228) est pourvue du tube de détection de la température de l'échantillon (229), l'extrémité inférieure de la plaque de support de rail de guidage (223) est reliée à la plateforme de stockage des grilles (230), et la plateforme de stockage des grilles (230) est configurée pour le placement des grilles biologiques (15) ; et la surface latérale de la plaque coulissante de support (222) est reliée à des chaînes, et la plaque coulissante de support (222) est reliée à la plaque de support (221) par une liaison coulissante vers le haut et vers le bas.

5. Le dispositif de refroidissement d'échantillons biologiques selon la revendication 1, **caractérisé en ce que** l'élément d'aspiration d'air (33) comprend une soufflante (331), une plaque grillagée (332) et un tube d'aspiration d'air (333), dans lequel
la partie supérieure de la soufflante (331) est reliée à la plaque grillagée (332), et la soufflante (331) est configurée pour être en communication avec le tube d'aspiration d'air (333).

6. Le dispositif de refroidissement d'échantillons biologiques selon la revendication 5, **caractérisé en ce que** l'élément chauffant (35) comprend un module de chauffage (351) et un tube d'admission d'air (352), dans lequel
le module de chauffage (351) est configuré pour être en communication avec le tube d'aspiration d'air (333), et le module de chauffage (351) est configuré pour être en communication avec le tube d'admission d'air (352).

7. Le dispositif de refroidissement d'échantillons biologiques selon la revendication 6, **caractérisé en ce que** le corps cylindrique de circulation (31) comprend une cavité d'accouplement (311), des tubes d'injection d'azote (312), des tubes d'éjection de gaz (313), un plateau de refroidissement guidé (314) et une cuve de circulation (315), dans lequel
le tube d'admission d'air (352) est configuré pour être en communication avec la cavité d'accouplement (311), la cavité d'accouplement (311) est disposée à l'intérieur de la cuve de circulation (315), les tubes d'injection d'azote (312) sont disposés autour de la cavité d'accouplement (311) et configurés pour être en communication avec la cavité d'accouplement (311), les tubes d'éjection de gaz (313) sont disposés sur les parties supérieures des tubes d'injection d'azote (312), les tubes d'éjection de gaz (313) sont configurés pour être en communication avec la cavité d'accouplement (311) par l'intermédiaire de conduites, plusieurs groupes de tubes d'éjection de gaz (313) et de tubes d'injection d'azote (312) sont disposés circonférentiellement, les tubes d'éjection de gaz (313) sont pourvus de trous ronds dans leur partie supérieure, les parties supérieures des tubes d'éjection de gaz (313) sont pourvues du plateau de refroidissement guidé (314), le plateau de refroidissement guidé (314) est pourvu de trous de forme maillée, la partie supérieure du plateau de refroidissement guidé (314) est pourvue de colonnes de grille, et les grilles biologiques (15) sont placées à l'intérieur de la cuve de circulation (315) par l'intermédiaire des colonnes de grille ; et plusieurs groupes de grilles biologiques (15) sont disposés à l'intérieur de la cuve de circulation (315).

8. Le dispositif de refroidissement d'échantillons biologiques selon la revendication 7, **caractérisé en ce que** le tube d'injection d'azote (312) est configuré pour être en communication avec la conduite d'alimentation en liquide (213).

9. Un système de refroidissement multifonctionnel, comprenant le dispositif de refroidissement d'échantillons biologiques selon l'une quelconque des revendications 1 à 8, et comprenant également un ensemble de transport (4), un ensemble de levage (5), un ensemble de bâti de support (6), un ensemble d'ouverture du couvercle (7), un ensemble rotatif d'alimentation en liquide (8), un ensemble de préhension de grille (9), un ensemble de pipette (10), une boîte de stockage temporaire d'échantillons (11), un lecteur de codes-barres (12) et une cuve de transfert (13), dans lequel
l'ensemble de bâti de support (6) est configuré pour supporter l'ensemble de transport (4), l'ensemble de levage (5) et l'ensemble de cavité (1), l'ensemble de transport (4) est configuré pour transporter la cuve de transfert (13), l'ensemble de levage (5) est configuré pour soulever la cuve de transfert (13) et pour être accouplé à l'ensemble de cavité (1), l'ensemble d'ouverture du couvercle (7) est configuré pour ouvrir un couvercle supérieur de la cuve de transfert (13), l'ensemble rotatif d'alimentation en liquide (8) est configuré pour ajouter le liquide dans la cuve de transfert (13), l'ensemble de pipette (10) est configuré pour aspirer l'azote excédentaire, l'ensemble de préhension de grille (9) est configuré pour saisir les grilles biologiques (15), la boîte de stockage temporaire d'échantillons (11) est configurée pour recevoir les grilles biologiques (15), et le lecteur de codes-barres (12) est configuré pour lire les étiquettes des grilles biologiques (15).

10. Le système de refroidissement multifonctionnel selon la revendication 9, **caractérisé en ce que** l'ensemble de cavité (1) comprend une plaque de support de fonctionnement (111) et un boîtier de cavité de fonctionnement (112), dans lequel la partie supérieure de la plaque de support de fonctionnement (111) est reliée au boîtier de cavité de fonctionnement (112), la plaque de support de fonctionnement (111) et le boîtier de cavité de fonctionnement (112) forment une cavité de fonctionnement, et la cavité de fonctionnement est pourvue intérieurement de l'ensemble d'ouverture du couvercle (7), de l'ensemble rotatif d'alimentation en liquide (8), de l'ensemble de préhension de grille (9), de l'ensemble de pipette (10), de la boîte de stockage temporaire d'échantillons (11) et du lecteur de codes-barres (12).

11. Le système de refroidissement multifonctionnel selon la revendication 10, **caractérisé en ce que** l'ensemble de bâti de support (6) comprend un bâti de support (61) et une plateforme de support (62), dans lequel l'extrémité supérieure du bâti de support (61) est reliée à la plaque de support de fonctionnement (111), et l'extrémité latérale du bâti de support (61) est pourvue de la plateforme de support (62).

12. Le système de refroidissement multifonctionnel selon la revendication 11, **caractérisé en ce que** l'ensemble de transport (4) comprend un chariot de transport (41) et un rail de guidage de transport (42), dans lequel
le rail de guidage de transport (42) est disposé sur la partie supérieure de la plateforme de support (62), et le chariot de transport (41) est relié de manière coulissante au rail de guidage de transport (42).

13. Le système de refroidissement multifonctionnel selon la revendication 12, **caractérisé en ce que** l'ensemble de levage (5) comprend un rail de guidage de levage (51), un moteur de levage (52), une vis-mère de levage (53), un coulisseau de levage (54), une plaque de levage en U (55) et une plaque de levage (56), dans lequel
le rail de guidage de levage (51) est relié verticalement et de manière fixe au bâti de support (61), le coulisseau de levage (54) est relié de manière coopérante à la vis-mère de levage (53), l'extrémité supérieure et l'extrémité inférieure du rail de guidage de levage (51) comportent des saillies destinées à supporter la vis-mère de levage (53), le moteur de levage (52) est configuré pour entraîner en rotation la vis-mère de levage (53) par l'intermédiaire d'une poulie, la surface latérale du coulisseau de levage (54) est reliée de manière fixe à la plaque de levage en U (55), la plaque de levage (56) est disposée sur le côté supérieur de la partie médiane du chariot de transport (41), la cuve de transfert (13) est disposée sur la surface d'extrémité supérieure de la plaque de levage (56), et la plaque de levage (56) est configurée pour monter et descendre par l'intermédiaire de la plaque de levage en U (55).

14. Le système de refroidissement multifonctionnel selon la revendication 13, **caractérisé en ce que** l'ensemble d'ouverture du couvercle (7) comprend une plaque de couvercle de porte (71), un moteur de couvercle de porte (72) et un axe de couvercle de porte (73), dans lequel
le moteur de couvercle de porte (72) est relié à l'axe de couvercle de porte (73), la plaque de couvercle de porte (71) est reliée à l'axe de couvercle de porte (73), l'axe de couvercle de porte (73) est configuré pour être supporté au moyen d'une base de support de couvercle de porte, et la base de support de couvercle de porte est reliée à la plaque de support de fonctionnement (111).

15. Le système de refroidissement multifonctionnel selon la revendication 14, **caractérisé en ce que** l'ensemble de préhension de grille (9) comprend des rails coulissants selon l'axe X (911), des rails coulissants selon l'axe Y (912), un moteur de préhension (913), un axe de préhension (914) et des pinces de préhension de grille (915), dans lequel
les rails coulissants selon l'axe Y (912) sont reliés au côté intérieur du boîtier de cavité de fonctionnement (112), les rails coulissants selon l'axe Y (912) sont disposés symétriquement, deux groupes de rails coulissants symétriques selon l'axe Y (912) sont reliés par l'intermédiaire des rails coulissants selon l'axe X (911), les rails coulissants selon l'axe X (911) sont reliés de manière coulissante aux rails coulissants selon l'axe Y (912), un côté des rails coulissants selon l'axe X (911) est pourvu du moteur de préhension (913), le moteur de préhension (913) est relié à l'axe de préhension (914) par l'intermédiaire d'une poulie, et la partie inférieure de l'axe de préhension (914) est reliée aux pinces de préhension de grille (915).

16. Le système de refroidissement multifonctionnel selon la revendication 15, **caractérisé en ce que** l'ensemble de pipette (10) comprend un vérin électrique, un moteur de pipette (102), une coupelle d'azote liquide (104), une vis-mère de pipette (105), une pipette (106) et une pointe de pipette (107), dans lequel
le vérin électrique est disposé sur un côté des rails coulissants selon l'axe X (911) éloigné du moteur de préhension (913), la surface latérale du vérin électrique est pourvue d'un rail de guidage du moteur, le moteur de pipette (102) est relié de manière coulissante au rail de guidage du moteur et la vis-mère de pipette (105) est reliée au moteur de pipette (102), l'extrémité inférieure du moteur de pipette (102) est reliée à la pipette (106), le côté extérieur de la pipette (106) est pourvu de la coupelle d'azote liquide (104), et l'extrémité inférieure de la pipette (106) est reliée à la pointe de pipette (107).

17. Le système de refroidissement multifonctionnel selon la revendication 16, **caractérisé en ce que** la boîte de stockage temporaire d'échantillons (11) est disposée sur la plaque de support de fonctionnement (111), les grilles biologiques (15) sont stockées dans la boîte de stockage temporaire d'échantillons (11), la partie supérieure de la boîte de stockage temporaire d'échantillons (11) est pourvue d'une seconde plaque de couvercle de porte, et un second moteur de plaque de couvercle de porte est configuré pour entraîner la seconde plaque de couvercle de porte afin d'ouvrir ou de fermer la boîte de stockage temporaire d'échantillons (11).

18. Le système de refroidissement multifonctionnel selon la revendication 17, **caractérisé en ce que** l'ensemble rotatif d'alimentation en liquide (8) comprend un moteur rotatif d'alimentation en liquide (81), une cuve d'alimentation en liquide (82) et un tube d'appoint de liquide (83), dans lequel
le moteur rotatif d'alimentation en liquide (81) est relié à la cuve d'alimentation en liquide (82) par l'intermédiaire d'une poulie, le tube d'appoint de liquide (83) est relié à la cuve d'alimentation en liquide (82), la cuve d'alimentation en liquide (82) est configurée pour être supportée par l'intermédiaire de l'arbre de support, et l'arbre de support est relié à la surface latérale intérieure du boîtier de cavité de fonctionnement (112).
